# EUROPÄISCHE PATENTSCHRIFT

(11) **EP 1 203 566 B1**
(45) Veröffentlichungstag und Bekanntmachung des Hinweises auf die Patenterteilung: **26.05.2004**
(21) Anmeldenummer: 01810967.8
(22) Anmeldetag: 03.10.2001
(51) Int. Cl.: A61B 10/00, A61B 17/00

(54) **Vorrichtung für die endo- und ektocervicale Probenahme**
Device for endo- and ectocervical sampling
Instrument d'échantillonnage endo- et ectocervical

(30) Priorität: 02.11.2000 CH 21372000
(43) Veröffentlichungstag der Anmeldung: 08.05.2002
(73) Patentinhaber: Sehhilfe Bern, Verein zur Unterstützung blinder und sehbehinderter Menschen im Kanton Bern, 3012 Bern (CH)
(72) Erfinder: Hofmann, Hansjörg, 3176 Neuenegg (CH)
(74) Vertreter: BOVARD AG - Patentanwälte

(56) Entgegenhaltungen:
- EP-A- 0 717 944
- WO-A-91/16855
- GB-A- 2 208 603
- US-A- 3 881 464
- US-A- 5 191 899
- PATENT ABSTRACTS OF JAPAN vol. 1998, no. 12, 31. Oktober 1998 (1998-10-31) & JP 10 179593 A (ASAHI OPTICAL CO LTD), 7. Juli 1998 (1998-07-07)

## Beschreibung

Die vorliegende Erfindung bezieht sich auf eine Vorrichtung für die zytologische Probenahme von Zellen aus dem inneren und dem äusseren Gebärmutterhals. Diese zytologischen Probenahmen oder Abstriche werden für die Krebsfrüherkennung angewendet.

Auf dem Fachgebiet ist eine Anzahl von Instrumenten bekannt, welche der endo- und ektocervicalen Probenahme dienen.

WO 91/16855 stellt den nächtsten Stand der Technik dar und beschreibt ein solches Instrument. Das Instrument besitzt ein Paar flexibler Bürstenbereiche am Ende eines Stiels, wobei die Bürstenbereiche eine Veloursoberfläche mit Fasern aufweisen. Diese flexiblen Bürstenbereiche sind über zwei Arme, die endständig an einem Griff angeordnet sind, mit einem Ende mit den flexiblen Bürstenbereichen gelenkverbunden. Die Bürstenanordnung weist im Wesentlichen die Konfiguration eines regelmässigen Vierecks auf. Der Bürstenbereich ist elastisch und flexibel ausgebildet, derart, dass er nach einer Verformung wiederum seine ursprüngliche Form annimmt.

GB 2 208 603 beschreibt eine Vorrichtung für die cervicale Probenahme, wobei in Fig. 3 eine Ausführungsform dargestellt ist, die einen an einem Stiel in der Längsrichtung befestigten Bürstenteil mit zwei im Wesentlichen rechtwinklig seitlich herausragenden Bürstenteilen aufweist. Die hier vorgesehenen Bürstenteile weisen endständig Spitzen auf, die unter Umständen zu Verletzungen führen können.

US 4,873,992 beschreibt ein cervicales Zytologiegerät, dessen bevorzugte Ausführungsform aus einem Griff und einer gebogenen Bürste besteht. Der Bürstenteil ist an drei Stellen gebogen, damit durch eine Rotation gleichzeitig eine Zellentnahme am innern und am äusseren Gebärmutterhals ermöglicht wird. Die vorgeschlagenen Bürsten sind zylindrisch angeordnet. Auch hier weist der in Griffrichtung herausragende Bürstenteil eine Spitze auf, die unter Umständen zu Verletzungen führen könnte.

US-A-3,881,464 beschreibt eine Vorrichtung für die Entnahme von cervikalen Zell- und Gewebeproben. Die Vorrichtung besteht aus einem kegelstumpfförmigen Bürstchen mit radial hervorstehenden Borsten, welche in einer Flüssigkeit löslich sind. Bei der beschriebenen Vorrichtung wird keine Schlaufe des Bürstenteils vorgeschlagen.

Im Weiteren beschreibt EP-A-0 717 944 ein Kosmetikbürstchen, das zur Auftragung von Mascara (Wimperntusche) auf die Wimpern dient. Das Bürstchen besitzt eine Seele umfassend eine Mehrzahl von zwischen wenigstens zwei miteinander verdrillten Drahtabschnitten festgelegten Borsten, wobei die Seele so ausgestaltet ist, dass sie eine ösenförmige Konfiguration bildet, die geeignet ist Mascara zum Auftragen auf die Wimpern aufzunehmen. Die gebildete Öse besitzt eine im wesentlichen längliche Ausgestaltung, wobei oval symmetrische und asymmetrisch tropfenförmige und auch zylinderförmige Ausgestaltungen erwähnt sind. Eine Ausgestaltung mit Ästen und eine Anpassung oder Verwendung für eine endo- und ektocervicale Probenahme sind jedoch nicht erwähnt.

Es ist Aufgabe der vorliegenden Erfindung, eine effiziente Vorrichtung für die zytologische Probenahme im Gebärmutterbereich zur Verfügung zu stellen, welche eine schonende Probenahme ohne Verletzungsgefahr erlaubt und deren Bürstenteil angepasst werden kann.

Es ist auch Aufgabe der vorliegenden Erfindung, ein Gerät zur Verfügung zu stellen, welches gemäss den in der Literatur beschriebenen Methoden eine nichttraumatische, wirksame und simultane Probenahme von endound ektocervicalen Epithelzellen erlaubt. Die Vorrichtung soll sowohl durch Ärzte wie auch durch paramedizinisches Personal einfach zu handhaben und kostengünstig herstellbar sein. Die durch die Vorrichtung gesammelten Zellproben sollen eine weitere Bearbeitung durch die klassische Pap-Methode erlauben, ebenso durch die empfindlichere Dünnschichtmethode. Die Vorrichtung soll, wenn die Dünnschichtmethode verwendet wird, eine zweckmässige und zuverlässige Fixierung der Zellen in einer minimalen Menge Fixierungslösung erlauben, da die Fixierungslösung einen bedeutenden Kostenfaktor darstellt. Der Bürstenteil soll leicht vom Griff entfernt werden können, damit er mit den gesammelten Zellen in einem geeigneten Gefäss mit Flüssigkeit transportiert werden kann.

Die Aufgabe wurde gelöst durch eine Vorrichtung für die zytologische Probenahme von Zellen aus dem innern und äusseren Gebärmutterhals. Die Vorrichtung besteht aus einem Griffstab mit einem Griff-Ende und einem Probenahme-Ende und einem Bürstenteil zur Probenahme. Der Bürstenteil ist am Probenahme-Ende angeordnet. Der Bürstenteil besteht aus einer einzigen länglichen verformbaren Spiraldraht-Bürste mit zwei Enden, die unter Bildung einer Schlaufe mit den beiden Enden am Probenahme-Ende des Griffstabes befestigt ist, wobei der Griffstab aus Kunststoff besteht. Die Vorrichtung ist dadurch gekennzeichnet, dass das Probenahme-Ende für die Aufnahme der Enden des Bürstenteils einen Klippverschluss aufweist, der die Enden in einer Bohrung oder einem Sackloch festhält. Vorteilhafterweise ist die verformbare Spiraldrahtbürste leicht elastisch.

Der Griffstab der erfindungsgemässen Vorrichtung weist vorzugsweise am Probenahme-Ende eine Sollbruchstelle auf, damit der Bürstenteil nach einer Probenahme zu seiner Auswertung abgebrochen werden kann.

Die beiden Enden des Bürstenteils sind zu ihrer Befestigung am Griffstab in der Regel frei von Borsten.

In einer besonders bevorzugten Ausführungsform ist die Schlaufe aus einer Spiraldrahtbürste derart geformt, dass sie drei Äste bildet, von welchen der mittlere Ast eine Verlängerung des Griffstabes bildet und die beiden seitlichen Äste im Wesentlichen rechtwinklig zur Stabrichtung, vorteilhaft in einem stumpfen Winkel bezüglich des Griffstabes, nach vorne herausragen.

Zur Anpassung an anatomische Gegebenheiten kann die Spiraldrahtbürste der erfindungsgemässen Vorrichtung in Abhängigkeit von der Position am Spiraldrahtkern unterschiedliche Borstenlängen aufweisen.

Der Griffstab besteht aus Kunststoff und ist am Probenahme-Ende für die Aufnahme der Enden des Bürstenteils mit einem integriertem Befestigungsmittel versehen, das ein Sackloch und einen Klippverschluss umfasst. Vorzugsweise hat das Sackloch zur Aufnahme der Enden des Bürstenteils einen länglichen oder ovalen Querschnitt. Dadurch kann die Herstellung erleichtert werden und gleichzeitig den Enden einen festen Sitz im Griffstab verliehen werden.

Der Griffstab ist vorteilhaft auf seiner Oberfläche in Längsrichtung gerillt, damit der Griff für die Rotation des Gerätes während der Probenahme erleichtert wird.

Die erfindungsgemässe Vorrichtung ist für den klassischen Pap-Test wie auch für das empfindlichere Dünnschichtverfahren geeignet. Die Vorrichtung weist ein Zellsammelmittel in Form einer Bürste auf, welches auf einem stabförmigen Griff montiert ist. Die Vorrichtung kann als Teil eines Komplettsystems bestehend aus Vorrichtung, Gefäss und gegebenenfalls auch Behandlungslösung, eingesetzt werden. Das nach der Probenahme verschlossene Gefäss ist für den Versand in ein diagnostisches Laboratorium geeignet. Mehrere Gefässe können z.B. als nummerierte Teströhrchen nebeneinander angeordnet sein, damit die Aufbewahrung und der Versand mehrerer identifizierbarer Proben erleichtert wird.

Die bisherige Erfahrung auf diesem Gebiet hat gezeigt, dass Bürstengeräte den Spaten und auch den Baumwollabstrichtupfern überlegen sind, da durch die Verwendung von Bürsten Zellen in genügenden Mengen gesammelt werden und auf einen Glasobjektträger übertragen werden können. Besser werden sie in eine Lösung übertragen und anschliessend unter einem Lichtmikroskop geprüft, wobei die Zellen nicht beeinträchtigt werden. Es ist wünschenswert, dass die Zellnahme gleichzeitig aus dem inneren und dem äusseren Gebärmutterhals vorgenommen werden kann. Wie erwähnt, sind entsprechende Geräte schon vorgeschlagen worden. Diese besitzen jedoch Nachteile bezüglich der Kosten oder aber der optimalen Positionierungsmöglichkeiten beziehungsweise der Anpassung. Es ist ebenfalls wichtig, dass ein Bruch eines solchen Instrumentes während der Probenahme vermieden wird.

Der Bürstenteil des Gerätes gemäss der vorliegenden Erfindung besteht aus einer Spiraldrahtbürste, vorzugsweise einer solchen aus rostfreien verdrehten Drähten, mit welchen radial herausragende Borsten befestigt sind, welche eine verformbare lineare Spiraldrahtbürste bilden. Diese lineare Bürste wird zu einer Schlaufe geformt, wobei die beiden Enden der Bürste ohne Borsten sind und dazu bestimmt sind, in solcher Weise im Griffstab befestigt zu werden, dass eine Loslösung ausgeschlossen ist. Die Schlaufe wird in eine kleeblattförmige Form gebracht, derart, dass drei Äste gebildet werden, nämlich ein mittlerer axialer Ast und zwei laterale Äste, welche nahezu rechtwinklig von der Griffrichtung nach aussen ragen. Für die Befestigung der beiden Enden des Bürstenteils besitzt der Griffstab eine Befestigungsvorrichtung. Diese kann eine Bohrung oder ein Sackloch in Kombination mit einem Klippverschluss sein, durch welche der Bürstenteil so befestigt werden kann, dass ein ungewolltes Loslösen unter den Anwendungsbedingungen mit Sicherheit ausgeschlossen ist. Vorzugsweise enthält die Befestigungsvorrichtung ein Sackloch mit einen länglichen Querschnitt, damit die Enden der Spiralbürsten mit optimalem Sitz nebeneinander Platz finden, wobei der verschlossene Klippverschluss ein Herausziehen des Bürstenteils verhindert, und zwar auch bei kombinierten Zugund Rotationsbewegungen.

Die Vorrichtung weist vorzugsweise am Griffstab eine Sollbruchstelle auf, damit der Bürstenkopf nach der Probenahme in ein Reagenzgefäss eingebracht werden kann, der Griffstab abgebrochen und das Gefäss verschlossen werden kann. Gewünschtenfalls kann das Gefäss mit einer Lösung versehen sein, welche dafür bestimmt ist, die Zellen für die spätere Untersuchung aufzunehmen. Der Durchmesser des Reagenzgefässes wird so ausgewählt, dass sich der Bürstenkopf verformt und im Gefäss leicht festgeklemmt wird. Die Vorrichtung kann in Sätzen mit zugehörenden Gefässen in den Handel gebracht werden. Beispielsweise Sätze mit 6, 12 oder 24 Teströhrchen, in Abhängigkeit des Verbrauchs des Arztes oder der Klinik. Alternativ kann das Gerät ebenfalls verwendet werden, um einen Abstrich auf einem Objektträger zu machen, welcher einem Test bzw. einer Untersuchung zugeführt werden kann. Die Sollbruchstelle am Griffstab muss so ausgelegt sein, dass ein unabsichtliches Zerbrechen ausgeschlossen ist.

Die Erfindung wird beispielsweise anhand der beiliegenden Zeichnungen näher erläutert. Es zeigt
Fig. 1 eine Ansicht eines erfindungsgemässen Gerätes,
Fig. 2 eine Ansicht des gleichen erfindungsgemässen Gerätes mit einem Teilschnitt,
Fig. 3 eine Ansicht des Querschnittes des Griffstabes eines erfindungsgemässen Gerätes und
Fig. 4 eine Ansicht der Stirnseite des Befestigungsmittels eines erfindungsgemässen Gerätes.

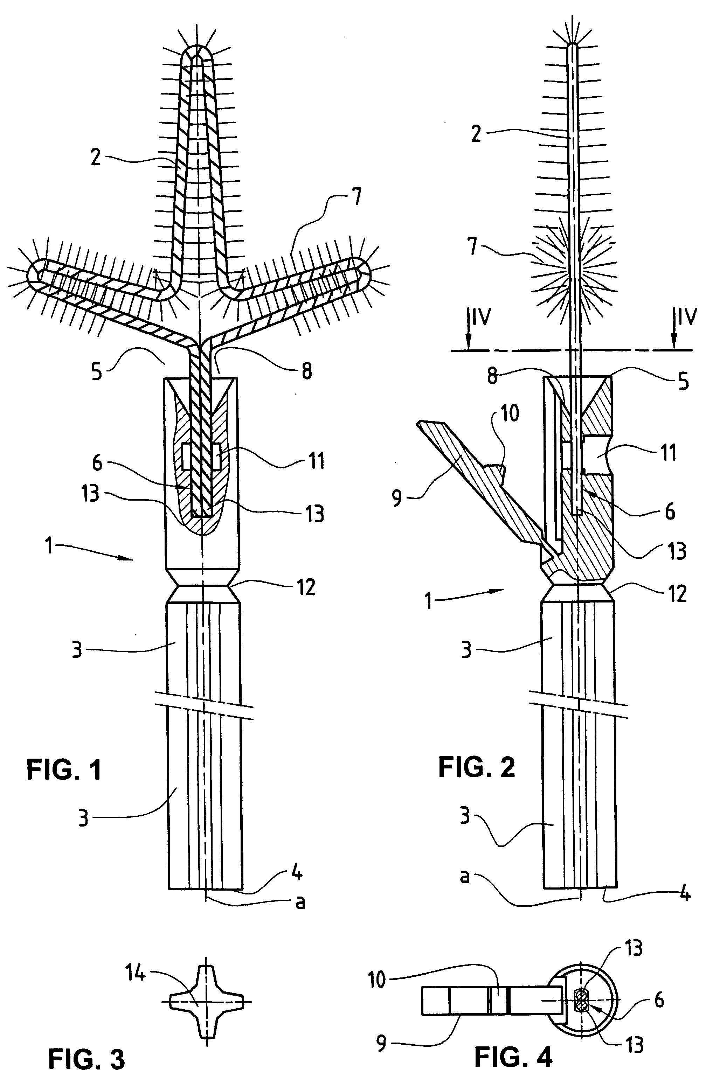

Fig. 1 zeigt eine erfindungsgemässe Vorrichtung 1 für die endo- und ektocervicale Probenahme. Die Vorrichtung besteht aus einem Bürstenteil 2 und einem Griffstab 3. Der Griffstab besitzt ein Griff-Ende 4 und ein Probenahme-Ende 5. Der Bürstenteil besteht aus verdrehten Drähten 2, in welche Borsten 7 aus Nylon eingedreht sind. Die Enden 13 des Bürstenteils sind frei von Borsten und sind für die Befestigung im Griffstab vorgesehen. Endständig weist der Griffstab eine trichterförmige Öffnung 8 auf, die in eine Bohrung oder ein Sackloch 6 mündet, welche der Aufnahme der Enden 13 dienen, die durch einen Klippverschluss (9, 10 in Fig. 2) fixiert werden können. Durch eine Queröffnung 11 kann ein Nocken eines Klipps eindringen und die Enden des Bürstenteils 13 unter leichtem Krümmen sicher halten. Der Griffstab 3 weist eine Sollbruchstelle 12 auf, welche nach der Probenahme zum Abbrechen des Griffstabes dient, sobald der Bürstenteil in ein Extraktions- oder Transportgefäss transferiert ist. Der Bürstenteil 2 ist eine Schlaufe, welche zu drei kleeblattartig angeordneten Ästen geformt ist. Der zentrale Ast, welcher aus dem Griffstab in Richtung der Achse a am Probenahme-Ende herausragt, dient der Probenahme im inneren Gebärmutterhals, während die seitlichen Äste der Probenahme am äusseren Gebärmutterhals dienen. Der Griffstab 3 weist eine solche Struktur auf, dass eine Rotation des Gerätes während der Probenahme durch die mit dieser Aufgabe betrauten Person erleichtert wird. Der Griffstab kann zur besseren Handhabung Längsrillen aufweisen.

Fig. 2 zeigt eine Zeichnung mit einem Teilschnitt des Griffstabes am Probenahme-Ende 5. Die Bezugszeichen entsprechen denjenigen in Fig. 1. Aus Fig. 2 geht eine zweckmässige Befestigungsweise des Bürstenteils 2 durch einen Klippverschluss 9 hervor. Die Enden 13 werden durch diesen Klippverschluss am Griffstab befestigt, indem sie durch die trichterförmige Öffnung 8 in die Bohrung bzw. das Sackloch 6 eingeführt werden. Beim Verschliessen greift ein am Klippverschluss 9 angeordneter Nocken 10 in die Queröffnung 11 ein. Der Nocken 10 übt im verschlossenen Zustand des Klippverschlusses 9 in der Öffnung 11 Druck auf die Enden 13 des Bürstenteils aus, wodurch diese leicht gebogen werden, was eine sichere Fixierung der Enden 13 bewirkt. Beim Bürstenteil 2 sind die unterschiedlichen Borstenlängen ersichtlich, so ist am äusseren Ende des mittleren Astes die Borstenlänge kürzer, als dies in der Nähe des Probenahme-Endes des Griffstabes und an den seitlichen Ästen der Fall ist. Die Ausgestaltung des Griffstabes mit Längsrillen kommt durch den kreuzförmigen Querschnitt 14 des Griffstabes zur Geltung.

Fig. 3 zeigt den Querschnitt des Griffstabes 3, der der Vorrichtung für die zytologische Probenahme einen verbesserten Griff bei der Anwendung verleiht.

Fig. 4 zeigt eine Ansicht der Stirnseite (Schnitt IV-IV in Fig. 2) des Probenahme-Endes des Griffstabes. Sichtbar ist die trichterförmige Öffnung mit dem zentralen Sackloch 6 zur Aufnahme der Enden 14 der Spiralbürste. Der mit dem Nocken 10 versehene Klippverschluss 9 ist in geöffnetem Zustand dargestellt. Der längliche Querschnitt 6 bietet gut Platz für beide Enden der Spiralbürsten und erlaubt mittels dem Klippverschluss ihre sichere Befestigung am Griffstab.

## Patentansprüche

1. Vorrichtung für die zytologische Probenahme von Zellen aus dem innern und äusseren Gebärmutterhals, bestehend aus einem Griffstab (3) mit einem Griff-Ende (4) und einem Probenahme-Ende (5), und einem Bürstenteil (2) zur Probenahme, welcher am Probenahme-Ende (5) angeordnet ist, wobei der Bürstenteil (2) aus einer länglichen verformbaren Spiraldrahtbürste mit zwei Enden (13) besteht, die unter Bildung einer Schlaufe mit den beiden Enden am Probenahme-Ende (5) des Griffstabes (3) befestigt ist, und der Griffstab (3) aus Kunststoff besteht, wobei das Probenahme-Ende (5) für die Aufnahme der Enden (13) des Bürstenteils einen Klippverschluss (9) aufweist, der die Enden (13) in einer Bohrung oder einem Sackloch (6) festhält.

2. Vorrichtung nach Anspruch 1, **dadurch gekennzeichnet, dass** die verformbare Spiraldrahtbürste (2) leicht elastisch ist.

3. Vorrichtung nach Anspruch 1 oder 2, **dadurch gekennzeichnet, dass** der Griffstab (3) beim Probenahme-Ende (5) eine Sollbruchstelle (12) aufweist, damit der Bürstenteil (2) mit dem Probenahme-Ende (5) nach einer Probenahme zu seiner Auswertung abgebrochen und in einem Gefäss transportiert werden kann.

4. Vorrichtung nach einem der Ansprüche 1 bis 3, **dadurch gekennzeichnet, dass** die Verformbarkeit des Bürstenteils (2) derart ist, dass seine Form den individuellen anatomischen Gegebenheiten angepasst werden kann.

5. Vorrichtung nach einem der Ansprüche 1 bis 4, **dadurch gekennzeichnet, dass** die beiden Enden (13) des Bürstenteils einander berührend zur Längsrichtung des Griffstabes (3) ausgerichtet und am Probenahme-Ende (5) unter Bildung einer in Richtung des Griffstabes (3) in eine Spitze auslaufende Schlaufe so befestigt sind, dass ein ungewolltes Loslösen ausgeschlossen ist.

6. Vorrichtung nach einem der Ansprüche 1 bis 5, **dadurch gekennzeichnet, dass** die Schlaufe derart geformt ist, dass sie drei Äste bildet, von welchen der mittlere Ast eine Verlängerung des Griffstabes bildet und die beiden seitlichen Äste in einem Winkel von 90° bis 45° zur Stabrichtung herausragen.

7. Vorrichtung nach einem der Ansprüche 1 bis 6, **dadurch gekennzeichnet, dass** die Spiraldrahtbürste des Bürstenteils einen Kern aus zwei spiralförmig verdrehten ineinander eingreifenden Drähten enthält, zwischen welchen Borsten aus Kunststoff in regelmässigen Abständen festgeklemmt sind.

8. Vorrichtung nach Anspruch 7, **dadurch gekennzeichnet, dass** die Spiraldrahtbürste in Abhängigkeit von der Position am Spiraldrahtkern unterschiedliche Borstenlängen aufweist.

9. Vorrichtung nach Anspruch 1, **dadurch gekennzeichnet, dass** am Probenahme-Ende ein Sackloch (6) mit einem länglichen Querschnitt vorgesehen ist, damit die Enden (13) des Bürstenteils passgenau nebeneinander Platz finden.

10. Vorrichtung nach einem der Ansprüche 1 bis 9, **dadurch gekennzeichnet, dass** der Griffstab (3) auf seiner Oberfläche in Längsrichtung gerillt ist, damit der Griff für die Rotation des Gerätes während der Probenahme erleichtert wird.

## Claims

1. Device for cytological sampling of cells from the endo- and ectocervix, consisting of a grip rod (3) with a grip end (4) and a sampling end (5), and a brush part (2) for sampling, which is disposed at the sampling end (5), the brush part (2) consisting of an elongated deformable spiral wire brush with two ends (13), which, while forming a loop with the two ends, is fixed at the sampling end (5) of the grip rod (3), and the grip rod (3) is made of plastic, the sampling end (5) having a clip closure (9) for receiving the ends (13) of the brush part, which clip closure firmly holds the ends (13) in a bore or in a blind hole (6).

2. Device according to claim 1, **characterised in that** the deformable spiral wire brush (2) is slightly elastic.

3. Device according to claim 1 or 2, **characterised in that** the grip rod (3) has a predetermined breaking point (12) at the sampling end (5) so that the brush part (2) with the sampling end (5) can be broken off for its evaluation after a sampling and can be transported in a container.

4. Device according to one of the claims 1 to 3, **characterised in that** the deformability of the brush part (2) is such that its shape can be adapted to the individual anatomical conditions.

5. Device according to one of the claims 1 to 4, **characterised in that** the two ends (13) of the brush part are aligned, touching each other, toward the lengthwise direction of the grip rod (3), and, while forming a loop tapering off to a point in the direction of the grip rod (3), are fastened at the sampling end (5) such that an undesired uncoupling is excluded.

6. Device according to one of the claims 1 to 5, **characterised in that** the loop is shaped such that it forms three branches, of which the middle branch forms a prolongation of the grip rod and the two lateral branches protrude at an angle of 90° to 45° with respect to the rod direction.

7. Device according to one of the claims 1 to 6, **characterised in that** the spiral wire brush of the brush part contains a core of two spiral-shaped engaging wires twisted into one another, between which bristles of plastic are firmly clamped at regular intervals.

8. Device according to claim 7, **characterised in that** the spiral wire brush has differing bristle lengths depending upon the position on the spiral wire core.

9. Device according to claim 1, **characterised in that** a blind hole (6) with an oblong cross-section is provided at the sampling end so that the ends (13) of the brush part have space next to one another in an exact-fitting way.

10. Device according to one of the claims 1 to 9, **characterised in that** the grip rod (3) is fluted in lengthwise direction on its surface, so that the grip is facilitated for rotation of the device during the sampling.

## Revendications

1. Dispositif pour le prélèvement cytologique d'échantillon de cellules endo- et ectocervicales du col de l'utérus se composant d'un bâtonnet de préhension (3) avec une poignée d'extrémité (4) et d'une extrémité de prélèvement (5) et d'une partie formant brosse (2) pour le prélèvement d'échantillon, laquelle est disposée sur l'extrémité de prélèvement (5), la partie formant brosse (2) se composant d'une brosse métallique en spirale allongée déformable avec deux extrémités (13) qui est fixée à l'extrémité de prélèvement (5) du bâtonnet de préhension (3) en formant une boucle avec les deux extrémités et le bâtonnet de préhension (3) se compose de matière plastique, l'extrémité de prélèvement (5) présentant pour la réception des extrémités (13) de la partie formant brosse une fermeture du type clip (9) qui maintient les extrémités (13) dans un perçage ou un trou borgne (6).

2. Dispositif selon la revendication 1, **caractérisé en ce que** la brosse métallique spiralée déformable (2) est légèrement élastique.

3. Dispositif selon la revendication 1 ou 2, **caractérisé en ce que** le bâtonnet de préhension (3) présente à la fin du prélèvement d'échantillon (5) un point de rupture obligatoire (12) afin que la partie formant brosse (2) avec l'extrémité de prélèvement (5) puissent être cassée après le prélèvement pour son analyse et être transportée dans un flacon.

4. Dispositif selon l'une des revendications 1 à 3, **caractérisé en ce que** la déformabilité de la partie formant brosse (2) est réalisée de sorte que sa forme puisse être adaptée aux conditions anatomiques individuelles.

5. Dispositif selon l'une des revendications 1 à 3, **caractérisé en ce que** les deux extrémités (13) de la partie formant brosse sont orientées mutuellement pour se toucher par rapport à l'axe longitudinal du bâtonnet de préhension (3) et sont fixées sur l'extrémité de prélèvement (5) en formant une bouche se terminant en pointe en direction du bâtonnet de préhension (3) de sorte qu'un détachement involontaire est exclu.

6. Dispositif selon l'une des revendications 1 à 4, **caractérisé en ce que** la boucle est réalisée de manière à former trois ramifications, la ramification centrale formant un prolongement du bâtonnet de préhension et les deux ramifications latérales faisant saillie en direction du bâtonnet en formant un angle de 90° à 45°:

7. Dispositif selon l'une des revendications 1 à 6, **caractérisé en ce que** la brosse métallique en spirale de la partie formant brosse comporte un noyau de deux fils engagés en forme de spirale l'un dans l'autre, entre lesquels des poils en matière plastique sont serrés à distance régulière.

8. Dispositif selon la revendication 7, **caractérisé en ce que** la brosse métallique en spirale présente des longueurs de poils différentes en fonction de la position sur le noyau de fil spiralé.

9. Dispositif selon la revendication 1, **caractérisé en ce que** sur l'extrémité de prélèvement, il est prévu un trou borgne (6) avec une section transversale allongée afin que les extrémités (13) de la partie formant brosse puissent avoir exactement la place l'une à côté de l'autre.

10. Dispositif selon l'une des revendications 1 à 9, **caractérisé en ce que** le bâtonnet de préhension (3) est nervuré sur sa surface dans le sens longitudinal afin que la poignée soit soulagée pour la rotation de l'appareil pendant le prélèvement.
